# FASCICULE DE BREVET EUROPEEN

(11) **EP 1 627 872 B1**
(45) Date de publication et mention de la délivrance du brevet: **15.08.2007**
(21) Numéro de dépôt: 05291234.2
(22) Date de dépôt: 08.06.2005
(51) Int. Cl.: C07D 271/12

(54) **Procédé de synthèse du sel de potassium du dinitrobenzofuroxanne**
Verfahren zur Herstellung von Kalium Salz von Dinitrobenzofuroxan
Process for the synthesis of the potassium salt of dinitrobenzofuroxane

(30) Priorité: 13.07.2004 FR 0407850
(43) Date de publication de la demande: 22.02.2006
(73) Titulaire: NEXTER Munitions, 78000 Versailles (FR)
(72) Inventeur: Coulouarn, Christophe, 18110 Pigny (FR)
(74) Mandataire: Célanie, Christian

(56) Documents cités:
- FR-A- 1 519 799
- US-A- 4 529 801
- GAUGHRAN R. J. ET AL.: "Contribution to the Chemistry of Benzfuroxan and Benzfurazan Derivatives" J. AM. CHEM. SOC., vol. 76, 1954, pages 2233-2236, XP002319175
- LI, YUFENG ET AL: "A new way to synthesize spherical potassium 4,6-dinitro-7-hydroxy-7- hydrobenzofuroxan (KDNBF)" HUOZHAYAO XUEBAO , 26(3), 53-56 CODEN: HUXUFP; ISSN: 1007-7812, 2003, XP009044480

## Description

Le domaine technique de l'invention est celui des procédés de synthèse du sel de potassium du dinitrobenzofuroxanne (ou KDNBF).

Ce sel est un explosif primaire bien connu de formule chimique brute (C₆HN₄O₆)K, H₂O qui est sensible aux chocs et au fil chaud. Il présente comme avantage sa faible toxicité car il ne comporte pas de métaux lourds tel que le plomb ou le mercure. Il est donc particulièrement recherché pour les applications civiles telles que la fabrication des inflammateurs pour systèmes de sécurité automobiles.

On connaît par le brevet FR1519799 un procédé de préparation de ce sel par la double décomposition entre un sel soluble du dinitrobenzofuroxanne (DNBF) de préférence un sel sodique (NaDNBF) et un sel de potassium donnant une solution neutre, de préférence le sulfate de potassium.

Le mélange est effectué lentement au goutte à goutte dans une solution maintenue à chaud (entre 60°C et 70°C).

Le sel de sodium du dinitrobenzofuroxanne (NaDNBF) est synthétisé aisément par réaction du dinitrobenzofuroxanne (DNBF) avec le carbonate de sodium (Na₂CO₃).

L'avantage du procédé décrit par FR1519799 est que le KDNBF obtenu a la forme d'une poudre formée de cristaux réguliers en plaquettes et dont la granulométrie est régulière et comprise entre 20 et 100 micromètres. Cette poudre a une densité de l'ordre de 0,6. Cette poudre coule bien ce qui assure une mise en oeuvre industrielle plus aisée qu'avec les KDNBF réalisés suivant les autres procédés de synthèse connus, tels que la réaction du DNBF avec une solution chaude de carbonate ou bicarbonate de potassium.

Elle a également une sensibilité au fil chaud bien reproductible d'une synthèse à l'autre en raison de la densité élevée et de la granulométrie modérée et régulière.

Un inconvénient de ce procédé est cependant qu'il requiert la synthèse et la séparation du sel de sodium (NaDNBF) avant la réalisation de la synthèse du KDNBF.

Il en résulte un temps de préparation total important de l'ordre de 10 heures.

C'est le but de l'invention que de proposer un procédé de préparation du KDNBF ne présentant pas de tels inconvénients.

Le procédé selon l'invention permet ainsi une synthèse plus rapide du KDNBF (gain de temps de 30% à 40%). Il permet néanmoins d'assurer l'obtention d'un KDNBF présentant des caractéristiques analogues à celles du KDNBF obtenu par le procédé décrit par FR1519799, notamment une forme de cristaux, une température d'inflammation et une granulométrie semblables.

Ainsi l'invention a pour objet un procédé de synthèse du sel de potassium du dinitrobenzofuroxanne (KDNBF), procédé comportant une étape de double décomposition entre un sel soluble du dinitrobenzofuroxanne (DNBF) et un sel neutre de potassium, procédé caractérisé en ce que la décomposition est réalisée directement dans le réacteur ayant servi à la synthèse du sel soluble du dinitrobenzofuroxanne, sans séparation complète de ce dernier de son milieu réactionnel.

Une telle disposition permet de réduire notablement le temps de préparation puisqu'il n'est plus nécessaire d'isoler le sel soluble de DNBF avant de procéder à la double décomposition telle que décrite par le brevet FR1519799.

Il n'était cependant pas évident qu'en procédant ainsi à la double décomposition directement dans le milieu réactionnel ayant permis la synthèse du sel soluble de DNBF le KDNBF obtenu aurait des caractéristiques de granulométrie, de Masse Volumique Apparente et de coulabilité de même niveau. La difficulté de cette synthèse était d'obtenir le même mode de cristallisation du KDNBF malgré la complexité du milieu réactionnel (présence de résidus des réactifs des deux synthèses successives). Le sel soluble du dinitrobenzofuroxanne préféré est le sel sodique du dinitrobenzofuroxanne (NaDNBF) car il présente une solubilité élevée. Le sel neutre de potassium préféré est le sulfate de potassium. Il serait également possible d'utiliser un chlorure de potassium.

Le procédé de synthèse comprend notamment les étapes suivantes :
- introduction de dinitrobenzofuroxanne (DNBF) dans une solution d'eau et d'acétone chauffée entre 20°C et 35°C, avec les proportions relatives de 8% à 12,5% en masse de DNBF pour 87,5% à 92% en masse d'une solution associant 80% à 100% d'eau pour 0% à 20% d'acétone, et mélange
- addition fractionnée de carbonate de sodium (Na₂CO₃) à cette solution dans les proportions en masse respectives de 2% à 7% de carbonate pour 93% à 98% de solution, et mélange pendant le temps nécessaire à la réaction,
- refroidissement.

Ces premières étapes conduisent à la synthèse du sel de sodium du dinitrobenzofuroxanne (NaDNBF).

Ce sel se trouve en solution dans son milieu réactif qui comprend essentiellement sous forme minoritaires des excédent de réactifs, et des produits comme des motifs (C₆H₂N₄O₆) ou du CO₂.

Le chauffage de la solution d'eau et d'acétone est réalisé grâce à un bain-marie thermostaté. Le DNBF utilisé est fourni commercialement à l'état humide. Son taux d'humidité varie entre 25 et 35%. La solution est agitée pendant toute la phase d'introduction du DNBF ainsi que pendant l'addition du carbonate de sodium.

Après refroidissement du NaDNBF ainsi synthétisé, on pourra directement procéder à l'introduction dans le milieu réactif d'un mélange chauffé entre 60°C et 70°C d'alcool polyvinylique et d'eau dans les proportions en masse respectives de 0,1% à 1% d'alcool pour 99% à 99,9% d'eau, puis à l'addition lente, progressive et sous agitation d'une solution de sulfate de potassium.

La solution de NaDNBF est maintenue pendant cette addition à une température comprise entre 20°C et 35°C.

On pourra également prévoir au moins une étape de décantation des cristaux de sel sodique du dinitrobenzofuroxanne (NaDNBF) et de séparation d'une partie des eaux mères avant d'introduire le mélange chauffé d'alcool polyvinylique et d'eau puis d'ajouter la solution de sulfate de potassium.

Cependant cette séparation des eaux mères conduit à un accroissement de la granulométrie de la poudre de KDNBF obtenue.

Afin de réduire la granulométrie de la poudre, on pourra avantageusement faire suivre cette étape de décantation des cristaux de NaDNBF et de séparation d'une partie des eaux mères par une étape de rinçage à l'eau déminéralisée suivie d'une autre étape de décantation et séparation partielle des eaux mères.

Afin d'améliorer encore la qualité du KDNBF obtenu, en augmentant notamment son taux en potassium (ce qui est un critère connu de la qualité du KDNBF, pour lequel généralement le taux de potassium doit être supérieur à 12,7%), on pourra avantageusement prévoir, après le rinçage à l'eau et la séparation partielle des eaux mères, au moins une étape de rinçage à l'éthanol, suivie d'une autre étape de décantation puis séparation partielle des eaux mères.

Dans tous les cas les étapes du procédé selon l'invention conduisent à l'obtention de cristaux de NaDNBF dans une solution d'une partie des eaux mères qui contient les résidus de réactions : réactifs en excédent, composés parasites en proportions minoritaires (produits de réactions entre les différents résidus).

Ces étapes sont ensuite suivies de la synthèse proprement dite du KDNBF par double décomposition.

Cette synthèse comprend les étapes suivantes :
- addition à cette solution d'un mélange chauffé entre 60°C et 70°C d'alcool polyvinylique et d'eau dans les proportions en masse respectives de 0,1% à 1% d'alcool pour 99% à 99,9% d'eau,
- chauffage et maintien de la solution de NaDNBF à une température comprise entre 60°C et 70°C,
- addition lente, progressive et sous agitation d'une solution de sulfate de potassium,
- refroidissement après réaction.

On pourra ensuite après refroidissement procéder à au moins une étape de rinçage à l'eau et/ou à éthanol puis à une étape de séchage du sel de potassium du dinitrobenzofuroxanne (KDNBF).

On a ainsi réalisé plusieurs synthèses de 200 g de KDNBF sans récupérer le NaDNBF.

Dans tous les cas le NaDNBF a été préparé suivant le procédé suivant :

### Procédé de préparation du NaDNBF

On introduit dans un réacteur 1800 ml (millilitres) d'eau déminéralisée à laquelle on ajoute 200 ml d'acétone. Le réacteur est placé dans un bain-marie thermostaté dont la température est réglée entre 20°C et 30°C. Un agitateur est introduit dans le réacteur et mis en marche.

On ajoute dans le réacteur 268 g (grammes) de DNBF humide (correspondant à un équivalent sec de 200 g de DNBF).

On mélange avec l'agitateur.

On ajoute de façon fractionnée 100 g de carbonate de sodium (Na₂CO₃) à la solution de DNBF ainsi préparée. Cette adjonction se fera sous la forme de 10 doses successives de 10 g de carbonate.

On laisse la réaction se poursuivre pendant une durée de 2 heures environ, tout en maintenant l'agitation.

On arrête alors le système de chauffe du réacteur et on place ce dernier dans un bain réfrigérant à environ 5°C. La réfrigération se poursuit sous agitation pendant au moins 20 minutes.

On laisse ensuite les cristaux de NaDNBF décanter dans le réacteur pendant au moins 5 minutes.

On a ensuite testé quatre modes opératoires différents :

### Exemple 1

On ne retire pas les eaux mères du réacteur et on conduit la double décomposition du NaDNBF directement dans le réacteur. On suit pour cela les étapes qui seront décrites par la suite

### Exemple 2

On retire les eaux mères surnageantes dans le réacteur en inclinant doucement celui ci. On retire ainsi environ 2000 ml de solution.

Puis on procède à la double décomposition du NaDNBF dans le réacteur. Là encore on suit pour cela les étapes qui seront décrites par la suite.

### Exemple 3

On retire les eaux mères surnageantes dans le réacteur en inclinant doucement celui ci. On retire ainsi environ 2000 ml de solution.

Puis on procède à un rinçage du NaDNBF en ajoutant dans le réacteur 200 ml d'eau distillée et en agitant pour homogénéiser.

On laisse ensuite décanter à nouveau les cristaux de NaDNBF et on retire une deuxième fois les eaux mères surnageantes dans le réacteur en inclinant doucement celui-ci. On retire ainsi environ 200 ml de solution.

Puis on procède à la double décomposition du NaDNBF dans le réacteur. Là encore on suit pour cela les étapes qui seront décrites par la suite.

### Exemple 4

Après les étapes de rinçage et décantation suivies à l'exemple 3, on procède à un deuxième rinçage à l'éthanol. On ajoute pour cela 200 ml d'éthanol sous agitation, puis après décantation des cristaux de NaDNBF on retire encore les eaux mères surnageantes en inclinant doucement le réacteur. On retire ainsi environ 200 ml de solution.

Puis on procède enfin à la double décomposition du NaDNBF dans le réacteur. Là encore on suit pour cela les étapes qui sont décrites ci-dessous.

### Procédé de synthèse du KDNBF par double décomposition du NaDNBF

Le procédé mis en oeuvre à chaque fois est le suivant :

On pèse au plus 30 g d'alcool polyvinylique que l'on dissout dans 1800 ml d'eau distillée. On réchauffe cette solution à environ 60°C et on l'ajoute dans le réacteur à la solution contenant le NaDNBF. L'alcool polyvinylique a pour fonction de favoriser la formation de cristaux de KDNBF grands et épais comme cela est décrit par le brevet FR1519799.

Le réacteur est maintenu alors à une température comprise entre 60°C et 70°C et la solution est sous agitation.

On prépare par ailleurs une solution de sulfate de potassium en versant 1350 ml d'eau déminéralisée dans un Becher puis en ajoutant 52 grammes de sulfate de potassium (K₂SO₄). La solution est chauffée à environ 60°C et agitée.

On raccorde une pompe péristaltique entre le Becher de sulfate de potassium et le réacteur contenant le NaDNBF et l'alcool polyvinylique. Cette pompe sera réglée de façon à assurer un débit d'addition du sulfate de l'ordre de 40 ml/minute.

La pompe est mise en marche. La réaction complète dure environ 60 minutes.

On refroidit ensuite fortement le réacteur sous agitation en portant le milieu réactionnel à environ 0°C pendant 30 minutes. Ce refroidissement intense a pour but de précipiter les cristaux de KDNBF en solution.

Après refroidissement on retire les eaux mères par filtrage sous vide, puis on lave une fois avec 300 ml d'eau déminéralisée. On filtre une nouvelle fois et on lave ensuite le KDNBF avec 300 ml d'éthanol (95°), on filtre le KDNBF sous vide et on laisse ensuite sécher sur le filtre.

Le dernier lavage à l'éthanol permet d'éviter l'agglomération du KDNBF en galette. Les cristaux restent bien isolés ce qui facilite la mise en oeuvre ultérieure de l'explosif. Par ailleurs la mise en oeuvre de la synthèse par lot de 200g permet d'éviter l'agglomération du KDNBF lors du séchage et améliore ainsi le rendement.

On a étudié les échantillons de KDNBF obtenus suivant les quatre exemples précédents et on les a comparés avec une composition de référence (KDNBF réf) obtenue avec le procédé connu, c'est à dire pour laquelle après décantation du NaDNBF on a procédé à son rinçage (eau, puis alcool), à sa filtration sous vide, puis à son séchage conduisant à sa récupération sous forme de poudre.

On a mesuré pour chaque échantillon obtenu le taux de potassium (%K), la densité (MVA), la température de décomposition du KDNBF (Td) et la granulométrie (D).

La température de décomposition du KDNBF a été mesurée par calorimétrie différentielle (DSC - ou Differential Scanning calorimetry). On provoque un échauffement progressif d'une masse de 0,2mg de KDNBF disposée dans un creuset en aluminium sous atmosphère d'azote. La température est augmentée régulièrement de 10°C par minute sur une gamme de 25°C à 500°C. On note la température Td à laquelle intervient la fusion du KDNBF. Cette analyse permet également de déterminer l'enthalpie échangée lors de la montée en température et de la transdormation du KDNBF. Cette température permet, suivant la forme du pic de fusion, de déterminer la présence d'impureté de façon qualitative. Le taux de potassium est un indicateur connu de la qualité du KDNBF. Il permet aussi de valider la reproductibilité de la synthèse. La mesure du taux de potassium a été effectuée par dosage gravimétrique d'un échantillon de KDNBF ayant subi une attaque acide (conduisant à la précipitation du potassium sous la forme de sulfate de potassium).

Les résultats de mesures sont consignés dans le tableau suivant.

| **Echantillon** | **%K** | **MVA** | **Td (°C)** | **D (micromètres)** |
|---|---|---|---|---|
| **KDNBF réf** | **13,60** | **0,45** | **219** | **80 à 100** |
| Exemple 1 | 13,24 | 0,39 | 214 | 100 |
| Exemple 2 | 13,33 | 0,51 | 215 | 250 |
| Exemple 3 | 13,26 | 0,54 | 216 | 80 |
| Exemple 4 | 13,53 | 0,35 | 217 | 50 à 100 |

On constate, ce qui n'était pas évident à priori, que les valeurs de sensibilité Td et de taux en potassium sont, avec le procédé selon l'invention, du même ordre qu'avec le procédé antérieur.

Un tel résultat n'était pas évident. En effet, rien ne permettait d'imaginer que le niveau de cristallisation du KDNBF et son taux de potassium seraient analogues en utilisant, non pas un NaDNBF pur, mais un mélange partiellement cristallisé et incorporé partiellement dans une eau mère dont la composition n'était pas a priori reproductible.

On constate qu'il est possible grâce au procédé selon l'invention d'obtenir une granulométrie du même ordre (inférieure ou égale à 100 micromètres) et avec un taux de potassium comparable, ce qui garantit des performances comparables au niveau d'un inflammateur à fil chaud.

On constate également l'influence des étapes de rinçage et décantation sur la granulométrie du KDNBF obtenu.

Il est ainsi plus avantageux du point de vue granulométrie de garder les eaux mères (exemple 1 : granulométrie 100 micromètres) que de retirer celles ci après décantation (exemple 2 : granulométrie 250 micromètres).

Les meilleurs résultats sont par contre ceux correspondant à l'exemple 4 pour lequel on a procédé après une première décantation et retrait des eaux mères à deux rinçages successifs, à l'eau puis à éthanol, avec à chaque fois une décantation et un retrait des eaux mères.

Le procédé selon l'invention permet de diminuer de façon importante la durée de la synthèse du KDNBF. Cette synthèse peut ainsi être effectuée en environ 6 heures contre 10 heures pour le procédé classique. Par ailleurs on évite ainsi la manipulation et le déplacement du NaDNBF pulvérulent qui est une substance sensible. On améliore donc la sécurité du procédé.

## Revendications

1. Procédé de synthèse du sel de potassium du dinitrobenzofuroxanne (KDNBF), procédé comportant une étape de double décomposition entre un sel soluble du dinitrobenzofuroxanne (DNBF) et un sel neutre de potassium procédé ***caractérisé en ce que*** la décomposition est réalisée directement dans le réacteur ayant servi à la synthèse du sel soluble du dinitrobenzofuroxanne sans séparation complète de ce dernier de son milieu réactionnel.

2. Procédé de synthèse selon la revendication 1, **caractérisé en ce que** le sel soluble du dinitrobenzofuroxanne est le sel sodique du dinitrobenzofuroxanne (NaDNBF).

3. Procédé de synthèse selon la revendication 2, **caractérisé en ce que** le sel neutre de potassium est le sulfate de potassium.

4. Procédé de synthèse selon la revendication 3, **caractérisé en ce qu**'il comprend les étapes suivantes :
- introduction de dinitrobenzofuroxanne (DNBF) dans une solution d'eau et d'acétone chauffée entre 20°C et 35°C, avec les proportions relatives de 8% à 12,5% en masse de DNBF pour 87,5% à 92% en masse d'une solution associant 80% à 100% d'eau pour 0% à 20% d'acétone, et mélange
- addition fractionnée de carbonate de sodium (Na₂CO₃) à cette solution dans les proportions en masse respectives de 2% à 7% de carbonate pour 93% à 98% de solution, et mélange pendant le temps nécessaire à la réaction,
- refroidissement.

5. Procédé de synthèse selon la revendication 4, **caractérisé en ce qu**'il comprend après refroidissement au moins une étape de décantation des cristaux de sel sodique du dinitrobenzofuroxanne (NaDNBF) et de séparation d'une partie des eaux mères.

6. Procédé de synthèse selon la revendication 5, **caractérisé en ce qu**'il comprend au moins une étape de rinçage à l'eau après décantation et séparation partielle, suivie d'une autre étape de décantation et séparation partielle des eaux mères.

7. Procédé de synthèse selon la revendication 6, **caractérisé en ce qu**'il comprend au moins une étape de rinçage à l'éthanol après rinçage à l'eau, décantation et séparation partielle, suivie d'une autre étape de décantation et séparation partielle des eaux mères.

8. Procédé de synthèse selon une des revendications 4 à 7, **caractérisé en ce que**, après refroidissement et décantation éventuelle de la solution de sel sodique du dinitrobenzofuroxanne (NaDNBF), il comprend les étapes suivantes :
- addition à cette solution d'un mélange chauffé entre 60°C et 70°C d'alcool polyvinylique et d'eau dans les proportions en masse respectives de 0,1% à 1% d'alcool pour 99% à 99,9% d'eau,
- chauffage et maintien de la solution de NaDNBF à une température comprise entre 60°C et 70°C,
- addition lente, progressive et sous agitation d'une solution de sulfate de potassium,
- refroidissement après réaction.

9. Procédé de synthèse selon la revendication 8, **caractérisé en ce qu**'après refroidissement on procède à au moins une étape de rinçage à l'eau et/ou à éthanol puis à une étape de séchage du sel de potassium du dinitrobenzofuroxanne (KDNBF).

## Claims

1. Process to synthesise potassium dinitrobenzofuroxan (KDNBF) salt, such process incorporating a double decomposition step between a soluble dinitrobenzofuroxan (DNBF) salt and a neutral potassium salt, **characterised in that** the decomposition is performed directly in the reactor having been used to synthesise the soluble neutral potassium salt without the full separation of the latter from its reaction medium.

2. Synthesis process according to Claim 1, **characterised in that** the soluble dinitrobenzofuroxan salt is dinitrobenzofuroxan sodium salt (NaDNBF).

3. Synthesis process according to Claim 2, **characterised in that** the neutral potassium salt is potassium sulphate.

4. Synthesis process according to Claim 3, **characterised in that** it comprises the following steps:
- introduction of dinitrobenzofuroxan (DNBF) into a solution of water and acetone heated to between 20°C and 35°C, with relative proportions of 8% to 12.5% in mass of DNBF for 87.5% to 92% in mass of a solution associating 80% to 100% water for 0% to 20% acetone, and mixture
- addition in fractions of sodium carbonate (Na₂CO₃) to this solution in the respective proportions in mass of 2% to 7% of carbonate for 93% to 98% of solution, and mixture for the time required to obtain the reaction,
- cooling.

5. Synthesis process according to Claim 4, **characterised in that** after cooling it comprises at least one step to decant the dinitrobenzofuroxan sodium salt (NaDNBF) crystals and separate part of the mother liquor.

6. Synthesis process according to Claim 5, **characterised in that** it comprises at least one water rinsing step after decantation and partial separation followed by another step of decantation and partial separation of the mother liquor.

7. Synthesis process according to Claim 6, **characterised in that** it comprises at least one ethanol rinsing step after the water rinsing step, decantation and partial separation, followed by another step of decantation and partial separation of the mother liquor.

8. Synthesis process according to one of Claims 4 to 7, **characterised in that**, after cooling and any decantation of the dinitrobenzofuroxan sodium salt (NaDNBF) solution, it comprises the following steps:
- addition to this solution of a mixture heated to between 60°C and 70°C of polyvinyl alcohol and water in the respective proportions in mass of 0.1% to 1% of alcohol for 99% to 99.9% of water,
- heating and maintenance of the NaDNBF solution at a temperature of between 60°C and 70°C,
- slow, gradual addition whilst stirring of a potassium sulphate solution,
- cooling after reaction.

9. Synthesis process according to Claim 8, **characterised in that** after cooling there is at least one water and/or ethanol rinsing step then a drying step of the potassium dinitrobenzofuroxan salt (KDNBF).

## Patentansprüche

1. Verfahren zur Synthese des Kaliumsalzes von Dinitrobenzofuroxan (KDNBF), wobei das Verfahren einen Schritt von Doppelumsetzung zwischen einem lösbaren Salz von Dinitrobenzofuroxan (DNBF) und einem neutralen Kaliumsalz umfasst, wobei das Verfahren ***dadurch gekennzeichnet ist, dass*** die Umsetzung direkt in dem Reaktor durchgeführt wird, welcher der Synthese des lösbaren Salzes von Dinitrobenzofuroxan gedient hat, ohne vollständiger Trennung dieses Letzteren von seinem reaktiven Milieu.

2. Syntheseverfahren nach Anspruch 1, **dadurch gekennzeichnet, dass** das lösbare Salz von Dinitrobenzofuroxan das Natriumsalz von Dinitrobenzofuroxan (NaDNBF) ist.

3. Syntheseverfahren nach Anspruch 2, **dadurch gekennzeichnet, dass** das neutrale Kaliumsalz das Kaliumsulfat ist.

4. Syntheseverfahren nach Anspruch 3, **dadurch gekennzeichnet, dass** es die folgenden Schritte umfasst:
- Einbringen von Dinitrobenzofuroxan (DNBF) in eine zwischen 20°C und 35°C aufgewärmte Lösung von Wasser und Aceton, mit den jeweiligen Verhältnissen von 8% bis 12,5% der Masse von DNBF für 87,5% bis 92% der Masse einer Lösung, die 80% bis 100% Wasser für 0% bis 20% von Aceton verknüpft, und Mischen
- stufenweise Zugabe von Natriumkarbonat (Na₂CO₃) in diese Lösung in den jeweiligen Massenverhältnissen von 2% bis 7% Karbonat für 93% bis 98% Lösung und Mischen während der für die Reaktion notwendigen Zeit,
- Abkühlen.

5. Syntheseverfahren nach Anspruch 4, **dadurch gekennzeichnet, dass** es nach Abkühlen wenigstens einen Schritt des Dekantierens der Natriumsalz-Kristalle von Dinitrobenzofuroxan (NaDNBF) und der Trennung eines Teiles der Mutterlaugen umfasst.

6. Syntheseverfahren nach Anspruch 5, **dadurch gekennzeichnet, dass** es wenigstens einen Schritt des Spülens mit Wasser nach Dekantieren und teilweiser Trennung umfasst, gefolgt von einem weiteren Schritt des Dekantierens und teilweiser Trennung der Mutterlaugen.

7. Syntheseverfahren nach Anspruch 6, **dadurch gekennzeichnet, dass** es wenigstens einen Schritt des Spülens mit Ethanol nach Spülen mit Wasser, Dekantieren und teilweiser Trennung umfasst, gefolgt von einem weiteren Schritt des Dekantierens und der teilweisen Trennung der Mutterlaugen.

8. Syntheseverfahren nach einem der Ansprüche 4 bis 7, **dadurch gekennzeichnet, dass** es nach Abkühlen und eventuellem Dekantieren der Natriumsalz-Lösung von Dinitrobenzofuroxan (NaDNBF) die folgenden Schritte umfasst:
- Zugabe zu dieser Lösung einer zwischen 60° und 70°C erwärmten Mischung von Polyvinylalkohol und Wasser in den jeweiligen Massenverhältnissen von 0,1% bis 1% Alkohol für 99% bis 99,9% Wasser,
- Erwärmen und Halten der Lösung von NaDNBF auf einer Temperatur zwischen 60°C und 70°C,
- langsame, schrittweise Zugabe unter Rühren einer Lösung von Kaliumsulfat,
- Abkühlen nach Reaktion.

9. Syntheseverfahren nach Anspruch 8, **dadurch gekennzeichnet, dass** nach Abkühlen wenigstens ein Schritt des Spülens mit Wasser und/oder mit Ethanol vorgenommen wird, dann ein Schritt des Trocknens des Kaliumsalzes von Dinitrobenzofuroxan (KDNBF).
